# EUROPÄISCHE PATENTSCHRIFT

(11) **EP 1 965 815 B1**
(45) Veröffentlichungstag und Bekanntmachung des Hinweises auf die Patenterteilung: **24.02.2016**
(21) Anmeldenummer: 06830277.7
(22) Anmeldetag: 01.12.2006
(51) Int. Cl.: A61K 35/64, A61P 17/02

(54) **ZUSAMMENSETZUNG ZUR WUNDHEILUNG, UMFASSEND SUBSTANZEN AUS DIPTEREN-LARVEN**
WOUND HEALING COMPOSITION COMPRISING SUBSTANCES OBTAINED FROM DIPTERA LARVAE
COMPOSITION FAVORISANT LA CICATRISATION DES PLAIES COMPRENANT DES SUBSTANCES PROVENANT DE LARVES DE DIPTERES

(30) Priorität: 20.12.2005 DE 102005061246
(43) Veröffentlichungstag der Anmeldung: 10.09.2008
(73) Patentinhaber: Alpha-Biocare GmbH, 40225 Düsseldorf (DE)
(72) Erfinder: D'HAESE, Jochen, 42551 Velbert (DE); MEHLHORN, Heinz, 41468 Neuss (DE); RUZICKA, Thomas, 80337 München (DE); SCHMIDT, Jürgen, 40221 Düsseldorf (DE); STEGE, Helger, 79111 Freiburg (DE)
(74) Vertreter: Gille Hrabal
(86) Internationale Anmeldenummer: PCT/EP2006/069210
(87) Internationale Veröffentlichungsnummer: WO 2007/071540

(56) Entgegenhaltungen:
- EP-A2- 0 182 278
- WO-A1-03/013557
- WO-A2-01/31033
- JP-A- 59 013 730
- HOROBIN A J ET AL: "MAGGOTS AND WOUND HEALING: AN INVESTIGATION OF THE EFFECTS OF SECRETIONS FROM LUCILIA SERICATA LARVAE UPON INTERACTIONS BETWEEN HUMAN DERMAL FIBROBLASTS AND EXTRACELLULAR MATRIX COMPONENTS" BRITISH JOURNAL OF DERMATOLOGY, Bd. 148, Nr. 5, Mai 2003 (2003-05), Seiten 923-933, XP001161231 ISSN: 0007-0963

## Beschreibung

Die vorliegende Erfindung betrifft eine Zusammensetzung zur Wundheilung, welche Substanzen aus Dipteren-Larven umfasst.

Bei der Heilung von Wunden spielen viele Faktoren und Prozesse eine Rolle. Sowohl bei akuten als auch chronischen Wunden verläuft die Heilung in drei aufeinanderfolgenden Phasen, der Exsudations-, der Proliferations- und schließlich der Reparationsphase. In der exsudativen Phase, die klinisch durch ein Wundödem und Wundschmerz charakterisiert ist, stehen Reaktionen mit vasokonstriktorischen und hämostaseologischen Effekten im Vordergrund. Hierbei werden Makrophagen, neutrophile Granulozyten und Lymphozyten angelockt. Durch sogenanntes Gewebedebridement werden Zelltrümmer und nekrotisches Gewebe durch Phagozytose entfernt. Die proliferative Phase der Wundheilung beginnt mit dem Einwandern von Fibroblasten und Gefäßendothelzellen. Die neu gebildete Gewebsmasse wächst an, und es erfolgt eine verstärkte Freisetzung von Zytokinen und Wachstumsfaktoren, die wiederum die Proliferation von Zellen und Neubildung von Gefäßen fördern. Die extrazelluläre Matrix wird in dieser Phase umgebaut, und letztlich entsteht ein gut kapillarisiertes Granulationsgewebe, das Makrophagen, Fibroblasten und Mastzellen enthält. In der abschließenden Epithelisierungs- und Reparationsphase wandern Keratinozyten in die Wunde ein. Während sich jetzt die Kapillarendichte reduziert, nimmt der Gehalt an Kollagen zu, wodurch sich die Narbe verfestigt.

Kommt es aber zu Störungen im Ablauf der komplexen Prozesse der Wundheilung, kann die Heilung beträchtlich verzögert werden. Dehnt sich dieser Vorgang auf mehr als 6 Wochen aus, so gilt dies als eine chronische Wundheilungsstörung. Störungen der Heilung treten häufig als Folge von Erkrankungen auf, insbesondere bei Diabetes mellitus und Immundepressionen, als Ergebnis einer Varikose oder bei bestimmten bakteriellen Infektionen. Zur Verbesserung der Wundheilung sind Maßnahmen zur Beschleunigung der Abläufe in den drei erwähnten Phasen der Wundheilung zu treffen. Insbesondere muss die Hauptursache der verzögerten Wundheilung - eine mangelnde Bildung von Granulationsgewebe - beseitigt werden, die entweder auf vermindertem Debridement der Wunde oder auf übermäßiger Bildung von Zelldetritus beruht.

### Stand der Technik

Als Maßnahmen zur Reinigung nekrotisch oder fibrinös belegter akuter oder chronischer Wunden eignen sich chirurgische Verfahren, jedoch werden dabei erneut Traumata gesetzt, die insbesondere bei chronischen Wunden zu einer erneuten Verzögerung der Wundheilung führen können. Bei infizierten Wunden sind diese Maßnahmen nicht oder nur mit Applikation von Antibiotika durchführbar. Beim Verfahren der Vakuumversiegelung werden die Wunden unter Sog okklusiv abgedeckt und die Beläge durch Unterdruck abgetragen. Dieses Verfahren ist effektiv, erfordert aber in der Regel eine Immobiliserung des Patienten. Pharmakologische Verfahren beruhen auf dem Einsatz von proteolytischen Enzymen. Bei den meisten vermarkteten Präparaten werden die Enzyme aus Bakterien, einzelligen Tieren oder Rindern gewonnen. Die Wirksamkeit der Präparate ist aber in der Praxis häufig gering, u.a. weil die Halbwertszeit der Enzyme zu kurz ist oder die abzubauenden Zielsubstanzen im nekrotischen Gewebe dem Wirkungsspektrum der Enzyme nicht entsprechen. Aufgrund geringer Wirksamkeit oder eines fehlenden Nachweises der Wirksamkeit ist eine Reihe kommerzieller Produkte inzwischen (Stand Dezember 2005) nicht mehr auf dem Markt erhältlich.

Als Maßnahme zur Wundheilung ist der Einsatz von lebenden Fliegenmaden der Art Lucilia sericata bekannt. Diese Art der Therapie, bei der Maden auf die Wunden verbracht werden, ist aus dar Volksmedizin seit Jahrhunderten bekannt - die Maden der Fliegen ernähren sich von nekrotischem Gewebe, nicht aber von gesundem Gewebe. Die Maden von Lucilia sericata lysieren das nekrotische Gewebe durch per os ausgeschiedene Substanzen und saugen dann den Zellbrei auf.

Die Probleme beim Einsatz von lebenden Maden bei der Wundheilung liegen in drei Bereichen. So ekeln sich viele Menschen vor den krabbelnden Maden, die zudem noch beim Festhalten in der Wunde mit ihren Mundhaken beachtliche Schmerzen bereiten können. Es ist schwierig, lebende Fliegenmaden frei von potentiellen Krankheitserregern wie Bakterien oder Pilzen zu züchten, um mikrobielle Infektionen des Patienten zu vermeiden. Auch beim Transport vom Labor zum Patienten müssen die Fliegenmaden keimfrei gehalten werden. Ein anderes Problem ergibt sich aus der Logistik der Maden, denn Fliegenmaden können auf diesem Stadium nicht "arretiert" werden, sondern wachsen weiter und verpuppen sich nach wenigen Tagen. Daher ist es nur schwer möglich, Larven von geeigneter Größe bei Ankunft eines entsprechenden Patienten in der Klinik einsatzfähig zu haben und für jede neue Applikation bereitzuhalten.

Aus DE 10149153 A1, DE 10138303 A1, DE 1 9901 134 C2 sowie DE 103 27 489 A1 ist bekannt, dass anstelle lebender Fliegenmaden auch aus den Maden gewonnene Sekrete oder Extrakte zur Therapie von Wunden verwendet werden können.

DE 103 27 489 A1 offenbart Homogenate, Extrakte und Bestandteile daraus aus Dipteren-Puppen und deren Verwendung zur Behandlung von Wunden. Zusammensetzungen aus Larven sind nicht offenbart. Weiter offenbart (1) weder das Molekulargewicht der extrahierten Substanzen noch deren Hitzestabilität. Im Gegensatz hierzu werden die Extrakte nach der Lehre dieses Dokuments unter ständiger Kühlung gewonnen und aufbewahrt.

JP 06 1 99898 offenbart einen Extrakt aus Stubenfliegen-Puppen, der Peptide mit einem Molekulargewicht von 3350 ± 900 Dalton umfasst. Die Hitzestabilität ist nicht erwähnt. Zudem werden die Extrakte gemäß (2) nicht zur Wundheilung, sondern als Tyrosinase-Inhibitor, Hautaufhellungsmittel, Konservierungsmittel für Lebensmittel oder Insektizide verwendet.

CN-A 1 231 31 (Derwent Abstracts accession number 2000-08779) offenbart ebenfalls kein Zusammensetzung zur Wundheilung, sondern einen Extrakt aus Fliegen-Larven oder -Puppen zur Verstärkung der Immunität und Tumorinhibierung. Das Molekulargewicht der Extraktbestandteile wird in diesem Dokument nicht definiert.

WO 2006/066619 ist ein nach dem Prioritätstag der vorliegenden Anmeldung veröffentlichtes Dokument. Das Dokument offenbart Extrakte aus Dipteren-Puppen, nicht aus Larven. Weiter sind weder das Molekulargewicht noch eine Hitzebeständigkeit der Extrakte offenbart. Im Gegenteil werden die Extrakte gemäß der Lehre dieses Dokuments ständig gekühlt.

WO 03/01 3557 offenbart zwar Extrakte aus Dipteren-Larven zur Wundheilung, aber weder das beanspruchte Molekulargewicht noch die Hitzebeständigkeit. Auch in diesem Dokument wird ausdrücklich gefordert, dass die Extrakte ständig gekühlt werden müssen.

Bei der Suche nach den wirksamen Substanzen der Madenextrakte wurde bereits in der Literatur vermutet, dass Allantoin, ein Produkt des Purinstoffwechsels, oder Harnstoff und/oder Ammonium die Wundheilung beschleunigen könnte (Robinson W: Stimulation of healing in non-healing wounds by allantoin occurring in maggot secretions and of wide biological distribution. J Bone Joint Surg 1953; 17:287-271. Robinson W: The healing properties of allantoin and urea discovered through the use of maggots in human wounds. Ann Rep Smithsonian Institution, Washington, DC US Government Printing Office. 1938, S. 451-460. Robinson W, Baker FC: The enzyme urease and occurrence of ammonia in maggot infected wounds. J Parasitol 1939; 25:1 49-1 55). Allantoin und Harnstoff werden in der jüngeren Zeit als kosmetische Wirkstoffe für zahlreiche Hautpflegeprodukte verwendet. Anhand der vielfachen Anwendung dieser Substanzen beim Menschen, insbesondere aus vielen dermatologischen Studien über eventuelle hautpflegende Eigenschaften dieser Substanzen kann jedoch als gesichert gelten, dass Allantoin, Harnstoff und Ammonium in Unterschied zu den Madenextrakten keine nennenswert wundheilende Wirkung haben.

Es wird aufgrund mehrerer Studien angenommen, dass Madenextrakte durch antimikrobielle Wirkung die Wundheilung begünstigen. Ein alkalischer pH-Wert der Wunden bei Besiedlung mit Maden, bedingt durch Ammonium oder Ammoniumbicarbonat, wurde als Grund für eine beschleunigte Wundheilung angesehen (Messer FC, McClellen RH: Surgical maggots. A study of their functions in wound healing. Journal of Laboratory and Clinical Medicine 1935; 20:1219-1226). Wunden können mit Bakterien infiziert sein, jedoch auch in vielen schlecht heilenden Wunden ist die Besiedlung mit pathogenen Keimen relativ gering (Falanga V: Wound healing and its impairment in the diabetic foot. Lancet 2005; 366:1 736-1 743). Es ist daher anzunehmen, dass der mikrobizide Effekt der Madensekrete nicht generell die beschleunigte Wundheilung erklären kann. Zudem wird bei dem erfindungsgemäßen Verfahren bei der Lyophilisation das Ammonium entfernt, so dass dieses hierbei für die Wundheilung keine Rolle spielen kann.

Die Reinigung der Wunde von abgestorbenen Zellen und Zelltrümmern, das Debridement, ist eine wichtige Phase zu Beginn des Heilungsprozesses. Zum Auflösen der Zelltrümmer und verschiedener Proteine der extrazellulären Matrix werden verschiedene Proteasen eingesetzt. WO 01/31033 beansprucht die Gewinnung einer bestimmten Protease mit einer Molekularmasse von etwa 25 KDa aus Lucilia sericata-Maden und deren Verwendung zur Wundheilung. Definierte Proteasen wurden auch zur Anwendung bei Patienten vorgeschlagen (Chambers L, Woodrow S, Brown AP. Degradation of extracellular matrix components by defined proteinases from the greenbottle larva Lucilia sericata used for the clinical debridement of non-healing wounds. Br. J. Dermatol 2003; 148:14-23). Kontrollierte klinische Studien zur Behandlung von Wunden mit Proteasen aus Dipteren liegen jedoch nicht vor und zur Zeit (Dezember 2005) sind keine kommerziellen Präparate mit Proteasen von Dipteren zur Behandlung von Wunden verfügbar.

Ein gravierendes Problem bei der Verwendung von Gesamtextrakten aus Dipteren für die Anwendung in Wunden besteht darin, dass eine Reihe von Proteinen in diesen Extrakten insbesondere bei wiederholter, längerfristiger Anwendung an Wunden Immunreaktionen induzieren können. Infolge dieser Immunreaktionen kann es zu Entzündungen der Wunden und einer generalisierten allergischen Reaktion des Körpers kommen DE 10327489 A1 beschreibt einen Extrakt aus Fliegenmaden, in dem jedoch viele immunogene Proteine mit gesundheitsschädigendem Potential enthalten sind.

Eine wichtige und bisher ungelöste Aufgabenstellung besteht nun darin, aus dem Extrakt von Dipteren solche Substanzen zu gewinnen, die erstens die Wundheilung stimulieren, die zweitens möglichst geringe immunologische Nebenwirkungen haben, die drittens frei von Kontamination mit Mikroorganismen hergestellt werden können und die viertens lagerstabil und als gut standardisierbares sowie dosierbares Medikament formuliert werden können.

Auf diesem Hintergrund ist die Aufgabe der Erfindung, eine Zusammensetzung zur Wundheilung bereitzustellen, das die oben erwähnten Nachteile nicht zeigt und die genannten Probleme löst.

### Offenbarung der Erfindung

Es wurde nun überraschend gefunden, dass nach vollständiger Entfernung jeglicher proteolytischer Aktivität von Fliegenmaden-Extrakten durch Behandlung mit Hitze, wobei die Proteasen denaturiert werden und desgleichen auch bei vorausgehender Ultrafiltration zur Entfernung aller Proteine mit einer Molekularmasse größer als 10 KDa, die Präparate bei Anwendung bei Patienten dennoch eine ausgezeichnet gute Wundheilung bewirken. Demnach sind Proteasen keineswegs wesentlich für die Wundheilung erforderlich -wie oftmals angenommen worden ist. Basierend auf diesem Ergebnis wurde die Erfindung vervollständigt.

Es wurde nun überraschenderweise gefunden, dass die erfindungsgemäßen Isolate, gewonnen aus Larven der Dipteren, die vorgenannten Nachteile der gegenwärtigen Therapiemaßnahmen bei der Wundheilung beheben können.

Die oben genannte Aufgabe wird gelöst durch eine Zusammensetzung zur Wundheilung nach Anspruch 1 . Bevorzugte Ausgestaltungen der Zusammensetzung sind in den Ansprüchen 2 bis 12 definiert. Die Erfindung betrifft die Verwendung einer erfindungsgemäßen Zusammensetzung zur Therapie von Wunden, sowie ein Verfahren zur Herstellung einer erfindungsgemäßen Zusammensetzung.

Die erfindungsgemäßen Extrakte aus Dipteren-Larven stellen eine signifikante Verbesserung der gegenwärtigen praktizierten Therapie mit lebenden Maden von Fliegen dar.

Es wurde gefunden, dass die wundheilenden Substanzen mittels Ultrafiltration durch eine Membran mit einer Ausschlussgrenze von 10.000 Dalton gewonnen werden können. Es zeigte sich weiterhin überraschend, dass bei einer zweiten Utrafiltration mit einer Membran mit einer Ausschlussgrenze von 500 Dalton die wirksamen Substanzen zurückgehalten werden. Eine beschleunigte Wundheilung ließ sich nur durch Anwendung der Fraktion erreichen, die Substanzen mit Molekularmassen zwischen 500 und etwa 10.000 Dalton enthält. Offensichtlich sind weder Proteine mit höherer Molekularmasse. u.a. Proteasen, noch sehr kleine organische oder anorganische Substanzen für die Wundheilung wichtig.

Es ist weiterhin möglich, die aufgereinigten Isolate mit Hitze zu behandeln, insbesondere bei 121 °C zu autoklavieren, und dadurch sicher keimfrei zu machen.

Es ist weiterhin möglich die Präparate ohne Wirkungsverlust zu trocknen, hierbei bevorzugt zu lyophilisieren, zu lagern und erst bei Bedarf zu rekonstituieren. Ein derartiges lagerfähiges Fertigpräparat stellt ein für den behandelnden Arzt oder für den Patienten stets verfügbares Medikament dar.

Geeignete Arten zur Herstellung der Präparate sind z.B. aus der Gattung Sarcophaga S. camaria, aus der Gattung Lucilia: L. sericata, P. regina aus der Gattung Phormia, C. erythrocephala aus der Gattung Calliphora und M. domestica aus der Gattung Musca. Diese Arten können im Labor leicht und in großer Menge für die Gewinnung der Extrakte gezüchtet werden.

Die gewonnenen Extrakte oder Isolate werden vor der Aufarbeitung in geeigneten Trägermedien, z.B. physiologischen Salzlösungen, sterilen Elektrolytlösungen, Albuminlösungen, Ölen oder Fetten aufbewahrt.

Die Erfindung betrifft auch ein Arzneimittel, gekennzeichnet durch einen wirksamen Gehalt an den erfindungsgemäßen Extrakten oder Bestandteilen daraus mit wundheilender Wirkung zusammen mit einem pharmazeutisch geeigneten physiologisch verträglichen Trägerstoff, Zusatzstoff und/oder anderen Wirk- und Hilfsstoffen. Wegen der pharmakologischen Eigenschaften eignen sich die erfindungsgemäßen Präparate zur Therapie von oberflächlichen oder tiefen chronischen wie akuten Wunden jeglicher Genese.

Unter dem Begriff "chronische und akute Wunden jeglicher Genese" werden beispielsweise Wunden verstanden wie Operationswunden, die gezielt oder ungewollt sekundär heilen sollen, Schnitt-, Stich-, Schürf-, Biss- oder Schussverletzungen sowie andere Wunden, die nicht primär mittels chirurgischer Naht oder einem primären Wundverschluss behandelt werden können. Ferner sind mit dem Begriff der akuten Wunden auch alle Wunden gemeint, die durch ein mikrobielle Infektion nicht primär abheilen können und alle Wunden, deren Manifestation 4 Wochen und weniger beträgt. Chronische Wunden sind alle Verletzungen, die mit der Aufhebung der Integrität des Epithels einhergehen und länger als 4 Wochen manifest sind. Insbesondere sind hiermit schlecht heilende Wunden auf der Basis eines Diabetes mellitus, einer Varikose oder einer Venenthrombose, eines rheumatischen Leidens, einer Vaskulitis, einer arteriellen Verschlusskrankheit, eines Leidens der Lymphgefäße, hämatologischer Erkrankungen und während oder nach Infektionen der Wunden gemeint.

Die Erfindung betrifft auch ein Verfahren zur Herstellung eines Arzneimittel, das dadurch gekennzeichnet ist, dass die erfindungsgemäßen Extrakte oder Bestandteile daraus mit wundheilender Wirkung mit einem pharmazeutisch geeigneten und physiologisch verträglichen Träger und gegebenenfalls weiteren geeigneten Wirk-, Zusatz- oder Hilfsstoffen in eine geeignete Darreichungsform gebracht werden.

Die Applikation der erfindungsgemäßen Arzneimittel erfolgt in der Regel topisch. Geeignete pharmazeutische Zusammensetzungen für die topische Anwendung auf der Haut liegen vorzugsweise als Salbe, Lösung, Suspension, Creme, Puder, liposomale oder olesomale Formulierungen, Gel, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Die erfindungsgemäßen Extrakte oder Enzymisolate sind im allgemeinen in einer Konzentration von 0,1 Gew.-% bis 100 Gew.-% der Zusammensetzung vorhanden, vorzugsweise von 1,0 Gew.-% bis 60 Gew.-%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten in geeigneter Weise die erfindungsgemäßen Extrakte oder Isolate in einer gegebenenfalls gepufferten wässrigen Lösung, gelöst und/oder dispergiert in einem Haftmittel oder dispergiert in einem Polymer. Eine geeignete Wirkstoff-Konzentration beträgt von etwa 0,1 Gew.-% bis 75 Gew.-%, vorzugsweise von 1 Gew.-% bis 70 Gew.-%. Als eine besondere Möglichkeit kann der Wirkstoff, wie beispielsweise in Pharmaceutical Research, 2: 318 (1986) beschrieben, durch Elektrotransport oder Iontophorese freigesetzt werden.

Die erfindungsgemäßen Extrakte können ferner auch auf die Wunde appliziert werden durch Wundauflagen aus Gaze, aus Alginaten, aus hydrokolloidalen Materialien, Schaumstoffen und/oder Silikonauflagen, die mit diesen Extrakten oder Enzymisolaten beschichtet, getränkt oder behandelt wurden und deshalb in der Lage sind, die Wirkstoffe in oder auf die Wunde bzw. Wundoberflöche abzugeben.

Geeignete feste oder galenische Zubereitungsformen sind beispielsweise Granulate, Pulver, Dragees, Tabletten, (Mikro-) Kapseln, Suppositorien, Sirupe, Säfte, Lösungen, Suspensionen, Emulsionen, Tropfen oder injizierbare Lösungen sowie Präparate mit protrahierter Wirkstoff-Freigabe, bei deren Herstellung übliche Hilfsmittel oder Trägerstoffe, wie Spreng-, Binde-, Überzugs-, Quellungs-, Gleit- oder Schmiermittel, Geschmackstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss-, oder Sesamöl, Polyethylenglykol und Lösungsmittel wie etwa steriles Wasser und ein- oder mehrwertige Alkohole wie Glycerin genannt.

Ferner können die erfindungsgemäßen Extrakte auch in galenischen Zubereitungen eingesetzt werden, denen zum Debridement geeignete Wirkstoffe, z.B. Enzyme, zugesetzt werden.

Einfache Beispiele sind die Verwendung eines Pulvers oder von Lyophilisaten, die in einer physiologischen Lösung (z.B. 0,9 %iger wässriger NaCl) aufgelöst werden. Bei ausreichender Stabilität kann die galenische Zubereitung auch eine Lösung sein.

Die Applikation der geeigneten pharmazeutischen Präparate erfolgt nach mechanischer Wundreinigung. Die mechanische Wundreinigung erfolgt beispielsweise durch ein Bad oder eine Spülung der Wunde mit Ringerlaktat. Die Wunde wird wahlweise nach Applikation oder erfindungsgemäßen Extrakte mittels hydrokolloidaler Wunddressings, oder mit Dipteren-Extrakt beschichteter Wundauflagen oder mittels selbstklebender Operations-Folie bedeckt. Verbandswechsel mit jeweils neuer Gabe der erfindungsgemäßen Extrakte oder Isolate erfolgen täglich.

### Beispiele

### Herstellung erfindungsgemäßer Präparate

Larven der Arten Musca domestica und/oder Calliphora erythrocephala, Lucilia sericata, Phormia regina, Sarcophaga camaria werden von frischem, oberflächlich abgeflammten und nicht kontaminiertem Pferdefleisch bzw. Pflanzenextrakt geerntet. Die dann gesammelten Maden werden in mehreren Waschschritten in sterilisierter, isoosmotischer Salzlösung äußerlich gesäubert. Danach werden die Maden zerdrückt, und der Inhalt wird in einem Trägermedium auf Eis gesammelt. Danach werden die Inhaltsstoffe homogenisiert. Dies geschieht in mehreren Schritten mittels Ultraschall oder mechanischer Homogenisation oder durch Zusatz von Lösungsmitteln. Nach Erhalt einer homogenen Flüssigkeit wird der Extrakt durch einen sterilen Filter mit einer Ausschlussgrenze von 10000 Dalton filtriert, z. B. mit Hilfe eines Vivacell 250-Filtrierapparats mit einer Membran aus Polyethersulfon 10.000 MWCO. Das Filtrat wird in einen Dialyseschlauch aus einer Cellulose-Membran mit einer Ausschlussgrenze von 500 Dalton gegeben und für 3 Tage gegen eine mehrfach gewechselte, 0,9 Gew.-% Natriumchlorid enthaltender, wässrige Lösung bei 4°C - zur Vermeidung mikrobieller Zerstörung - dialysiert. Die im Dialyseschlauch zurückgehaltene Lösung wird in geeigneten Gefäßen aliquotiert oder auf geeignete Wundauflagen durch Aufsprühen verteilt. Die Präparate werden dann durch Erhitzen auf 100°C für 30 Minuten oder bevorzugt durch Autoklavieren bei 121°C unter Druck sterilisiert. Die Präparate sind dann unter Kühlung lagerfähig. Bevorzugt werden die Präparate jedoch unter einer Sterilbank getrocknet bzw. lyophilisiert, um diese ohne Kühlung lagerfähig zu machen.

### Beispiel 1 : Herstellung eines Präparat aus einer Fliegenart

1.500 Fliegenmaden der Art Lucilia sericata wurden aus den Produktionskäfigen entnommen und im Kühlschrank gesammelt, bis die Anzahl erreicht ist. Dann wurden sie mechanisch homogenisiert und zunächst in einer Kühlzentrifuge für 2 Stunden zentrifugiert. Der Überstand wurde dann noch für 1 2 Stunden mit einer Beckman-Ultrazentrifuge zentrifugiert. Der Überstand wurde dann wie oben beschrieben ultrafiltiert, dialysiert, autoklaviert und in Portionen von 1 ml aufbewahrt. Zum Einsatz kommen 0,2 Milliliter pro 10 cm² Fläche der Wunde.

### Anwendungsbeispiele zur Wundbehandlung bei Patienten

### Anwendungsbeispiel 1

Ein 87-jähriger Patient mit einem posttrombotischen Syndrom, chronisch venöser Insuffizienz und seit drei 3 Jahre bestehendem Ulcus cruris venosum am linken Unterschenkel. Das Ulcus war während dieser Zeit trotz moderner Wundbehandlung nicht abgeheilt. Es bestanden bei Einleitung der Therapie großflächige Ulzerationen und Nekrosen. Es wurde ein Präparat angewendet, das wie in Beispiel 1 aus Maden der Art Lucilia sericata hergestellt und in flüssiger Form bei 4 Grad Celsius aufbewahrt worden war. Die tägliche Anwendung von 0,2 ml pro 10 cm² Fläche der Wunde führte innerhalb von 10 Tagen zu einem kompletten Debridement und beginnender Epithelisierung. Nach 8 Wochen Therapie unter zusätzlicher Kompression kam es zu einer kompletten Abheilung.

### Anwendungsbeispiel 2

Eine 72-jährige Patientin mit chronisch venöser Insuffizienz Grad IV, das Ulcus bestand seit 18 Monaten und wies chronische Fibrinbeläge auf. In der Vergangenheit konnten weder Hydrokolloidverbände noch PU-Schäume das Auftreten von Belägen verhindern. Es kam nicht zu einer Abheilung. Zur Herstellung des Präparats wurden ultrafiltierte und dialysierte Extrakte aus Maden der Art Musca domestica in einer Dosis von 30 Mikroliter pro cm² Wundauflage aufgetragen, dann autoklaviert, unter einer sterilen Arbeitsbank getrocknet und schließlich steril verpackt. Initial erfolgte die Behandlung durch einen täglichen Wechsel der Wundauflage, später jeden zweiten Tag. Unter der Therapie kam es zu einem kompletten Debridement und einer Abheilung des Ulcus innerhalb von 14 Wochen. Nebenwirkungen wurden nicht beobachtet. Nach 8 Monaten wurde bei der Patientin erneut ein Ulcus behandlungsbedürftig. Es wurden auch in diesem Fall mit einer Wundauflage therapiert, die mit getrocknetem Madenextrakt beschichtet war. Es erfolgte eine Ausheilung in 9 Wochen, ohne dass Nebenwirkungen in Form entzündlicher Immunreaktionen auf den erfindungsgemäßen Madenextrakt zu beobachten waren.

## Patentansprüche

1. Zusammensetzung zur Anwendung in einem Verfahren zur Wundheilung, bestehend aus einem Gemisch von bei 100 °C hitzestabllen Substanzen mit Molekularmassen zwischen 500 und 10.000 Dalton aus Dipteren-Larven, und einem pharmazeutisch geeigneten und physiologisch verträglichen Träger, und gegebenenfalls geeigneten Zusatzstoffen, geeigneten Hilfsstoffen, Mitteln zur Konservierung, mikrobiziden oder antioxidativen Substanzen, sowie gegebenenfalls zusätzlich einer oder mehreren Proteasen, die zum Debridement der Wunde das nekrotische Gewebe auflösen, in einer geeigneten Darrelchungsform.

2. Zusammensetzung zur Anwendung in einem Verfahren zur Wundheilung nach Anspruch 1, **dadurch gekennzeichnet, dass** sie in Form einer Wundauflage aus Gaze, aus Alginaten, aus kolloidalen Materialien, aus Schaumstoffen oder aus Silikonauflagen vorliegt.

3. Zusammensetzung zur Anwendung in einem Verfahren zur Wundheilung nach Anspruch 1 oder 2, **dadurch gekennzeichnet, dass** es sich um ein Pflaster handelt.

4. Zusammensetzung zur Anwendung in einem Verfahren zur Wundheilung gemäß Anspruch 1 oder 2, erhältlich aus Larven der Fliegengattungen Musca, Calliphora, Phormia, Sarcophaga oder Lucilia oder Gemischen von Larven von zwei oder mehr Arten.

5. Zusammensetzung zur Anwendung in einem Verfahren zur Wundheilung gemäß Anspruch 1, 2 oder 3, **dadurch gekennzeichnet, dass** sie aus den Arten Musca domestica, Calliphora erythrocephala, Sarcophaga canaria, Phormia regina, Lucilia sericata und/oder Lucilia caesar stammt.

6. Zusammensetzung zur Anwendung In einem Verfahren zur Wundheilung gemäß den Ansprüchen 1, 4, und/oder 5, **dadurch gekennzeichnet, dass** sie als galenische Zusammensetzung zur topischen Anwendung auf der Haut vorliegt, z.B. Salbe, Lösung, Suspension, Creme, Puder, liposomale oder oleosomale Formullerung, Gel, Lotion, Paste, Spray, Aerosol oder Öl.

7. Zusammensetzungen zur Anwendung In einem Verfahren zur Wundheilung nach einem oder mehreren der Ansprüche 1 bis 6 zur Therapie von oberflächlichen oder tiefen, chronischen und akuten Wunden jeglichen Ursprungs.

8. Verfahren zur Herstellung einer Zusammensetzung zur Anwendung in einem Verfahren zur Wundheilung gemäß einem oder mehreren der Ansprüche 1 bis 7, **dadurch gekennzeichnet, dass** Dipteren-Larven mechanisch, durch Zusatz von Chemikalien und/oder durch Ultraschall homogenisiert werden und anschließend einer Ultrafiltration zur Entfernung aller Proteine mit einer Molekularmasse größer als 10 kDa und einer Ultrafiltration mit einer Ausschlussgrenze von 500 Da unterzogen werden und anschließend jegliche proteolytische Aktivität durch Behandlung mit Hitze entfernt wird.

9. Verfahren gemäß Anspruch 8, **dadurch gekennzeichnet, dass** die Trennung von unlöslichen Bestandteilen und hochmolekularen Substanzen durch Zentrifugation, Ultrazentrifugation, Phasentrennung, Ultrafiltration und/oder chromatographische Trennung erfolgt.

10. Verfahren gemäß einem oder mehreren der Ansprüche 8 bis 10. **dadurch gekennzeichnet, dass** eine Hitzebehandlung bei 60 bis 100 °C durchgeführt wird oder die Zusammensetzung autoklaviert wird.

11. Verfahren gemäß einem oder mehreren der Ansprüche 8 bis 10, **dadurch gekennzeichnet, dass** eine Sterilfiltration mit einem Filter mit einem Durchmesser der Poren von 0,1 µm bis 0,4 µm erfolgt.

12. Verfahren gemäß einem oder mehreren der Ansprüche 8 bis 10, **dadurch gekennzeichnet, dass** das Präparat getrocknet und lyophilisiert und dadurch haltbar gemacht wird.

## Claims

1. A composition for use in a process for wound healing consisting of a mixture of substances heat-resistant at 100 °C having molecular masses from between 500 and 10.000 Dalton, from dipterous larvae, and a pharmaceutically suitable carrier and eventually suitable excipients, agents for preservation, microbicidal substances or antioxidants, as well as eventually, in addition, one or more proteases, which dissolve the necrotic tissue for debridement of the wound, in a pharmaceutically suitable form.

2. The composition for use in a process for wound healing according to claim 1, **characterized in that** it is present in a form of a wound dressing of gauze, alginates, colloidal materials, synthetic foams, or of silicone dressings.

3. The composition for use in a process for wound healing according to claim 1 or 2, **characterized in that** it is a Band-Aid.

4. The composition for use in a process for wound healing according to claim 1, 2, obtainable from larvae of the genus of flies of Musca, Calliphora, Phormia, Sarcophaga or Lucilla or mixtures of larvae of two or more genera.

5. The composition for use in a process for wound healing according to claim 1, 2 or 3, **characterized in that** it is derived from the genera of Musca domestica, Calliphora erythrocephaia, Sarcophaga canaria, Phormia regina, Lucilla sericata and/or Lucilla caesar.

6. The composition for use in a process for wound healing according to claim 1, 4 and/or 5, **characterized in that** it is present as a galenic composition for topical application onto the skin, e.g. solution, suspension, crème, powder, liposomal or oleosomal formulation, gel, lotion, paste, spray, aerosol or oil.

7. The composition for use in a process for wound healing according to one or more of the claims 1 to 6, for therapy of superficial wounds or deep, chronical and severe wounds of any origin.

8. A process for the production of a composition for use in a process for wound healing according to one or more of the claims 1 to 7, **characterized in that** dipterous larvae are mechanically homogenized by adding chemicals, and/or by ultrasound and are subsequently submitted to ultrafiltration to remove any of the proteins with a molecular mass of greater than 10 Da and to ultrafiltration having an exclusion limit of 500 Da and subsequently any proteolytic activity is removed by treatment with heat.

9. The process according to claim 8, **characterized in that** the separation of insoluble ingredients and of high-molecular weight substances is performed by centrifugation, ultracentrifugation, phase separation, ultrafiltration and/or chromatographic separation.

10. The process according to one or more of the claims 8 to 9, **characterized in that** heat treatment is performed at 60 to 100 °C or **in that** the composition is autoclaved.

11. The process according to one or more of the claims 8 to 10, **characterized in that** sterile filtration is performed with a pore diameter of 0,1 µm to 0,4 µm.

12. The process according to one or more of the claims 8 to 10, **characterized in that** the preparation is dried and lyophilized and thereby preserving it.

## Revendications

1. Composition pour l'utilisation dans d'un procédé pour la cicatrisation constituée d'un mélange de substances résistantes à la chaleur à 100°C ayant des masses moléculaires entre 500 et 10.000 Dalton des larves de diptères et d'un support pharmaceutiquement convenable et éventuellement des excipients convenables, des agents pour la préservation, des substances microbicides ou antioxydants, ainsi que éventuellement en outre d'un ou plusieurs protéases, qui, pour le débridement de la plaie, résolvent le tissue nécrotique, dans une forme pharmaceutique convenable.

2. Composition pour l'utilisation dans d'un procédé pour la cicatrisation selon la revendication 1 **caractérisée en ce qu'**elle est présente dans une forme d'une compresse de gaze, d'alginates, de matériaux colloïdes, de mousses synthétiques ou de compresses de silicone

3. Composition pour l'utilisation dans d'un procédé pour la cicatrisation selon la revendication 1 ou 2 **caractérisée en ce qu'**elle est un pansement.

4. Composition pour l'utilisation dans d'un procédé pour la cicatrisation selon la revendication 1 ou 2, accessible à partir de larves des genres de mouches Musca, Calliphora, Phormia, Sarcophaga ou Lucilla ou de mélanges de larves de deux ou plusieurs genres.

5. Composition pour l'utilisation dans d'un procédé pour la cicatrisation selon la revendication 1, 2 ou 3, **caractérisée en ce qu'**elle est dérivée des genres Musca domestica, Calliphora erythrocephaia, Sarcophaga canaria, Phormia regina, Lucilla sericata et/ou Lucilla caesar.

6. Composition pour l'utilisation dans d'un procédé pour la cicatrisation selon la revendication 1, 4 et/ou 5, **caractérisée en ce qu'**elle est présente en tant que composition galénique pour l'application topique sur la peau, ex. pommade, solution, suspension, crème, poudre, formulation liposomale ou oléosomale, gel, lotion, pâte, spray, aérosol ou huile.

7. Composition pour l'utilisation dans d'un procédé pour la cicatrisation selon l'une ou plusieurs des revendications 1 à 6, pour la thérapie des plaies superficielles ou des plaies profondes, chroniques et aiguës n'importe quel origine.

8. Procédé pour la production d'une composition pour l'utilisation dans d'un procédé pour la cicatrisation selon l'une ou plusieurs des revendications 1 à 7, **caractérisé en ce que** des larves de diptères sont homogénéisées mécaniquement, en ajoutant des produits chimiques, et/ou par ultrason et sont soumises ensuite à une ultrafiltration afin d'enlever de toutes les protéines avec une masse moléculaire supérieure à 10 Da et à une ultrafiltration avec un seuil de coupure de 500 Da et ensuite toute activité protéolytique est enlevée par traitement par la chaleur.

9. Procédé selon la revendication 8, **caractérisé en ce que** la séparation des ingrédients insolubles et des substances hautement moléculaires est mise en oeuvre par centrifugation, ultracentrifugation, séparation en phase, ultrafiltration and/or séparation chromatographique.

10. Procédé selon l'une ou plusieurs des revendications 8 à 9, **caractérisé en ce qu'**un traitement à la chaleur est réalisé à 60 jusqu'à 100°C ou **en ce que** la composition est autoclavée.

11. Procédé selon l'une ou plusieurs des revendications 8 à 10, **caractérisé en ce qu'**une filtration stérile est mise en oeuvre avec un diamètre de pores de 0,1 µm à 0,4 µm.

12. Procédé selon l'une ou plusieurs des revendications 8 à 10, **caractérisé en ce que** la préparation est séchée et lyophilisée et de ce fait est conservée.
